# EUROPEAN PATENT SPECIFICATION

(11) **EP 1 199 370 B1**
(45) Date of publication and mention of the grant of the patent: **21.04.2004**
(21) Application number: 00122707.3
(22) Date of filing: 18.10.2000
(51) Int. Cl.: C12Q 1/42, C07K 7/06, G01N 33/573

(54) **Process for detecting dephosphorylation of phosphorylated threonine or serine by phosphatase activity**
Verfahren zur Auffindung von Dephosphorylierung phosphorylierte Threonin oder Serin durch Phosphataseaktivität
Méthode pour détecter la déphosphorylation de thréonine ou sérine phosphorylées par l'activité de la phosphatase

(43) Date of publication of application: 24.04.2002
(73) Proprietor: Evotec OAI AG, 22525 Hamburg (DE)
(72) Inventor: Krämer, Joachim, Dr., 25474 Ellerbek (DE); Peiker, Christiane, 25469 Halstenbek (DE); Henco, Karsten, Dr., 40699 Erkrath (DE)
(74) Representative: Hertz, Oliver, Dr.

(56) References cited:
- EP-A- 0 874 052
- WO-A-93/10461
- WO-A-99/29894
- US-A- 5 814 507

## Description

The present invention relates to a process for detecting dephosphorylation of phospho-serine or phospho-threonine (hereinafter also "phospho-serine/-threonine") by the activity of a phosphatase in an immunoassay. The invention relates further to a kit for carrying out the assay and to a luminescently labelled ligand.

Reversible protein phosphorylation is an important mode of regulation of cellular processes. It is now apparent that protein phosphatases play in comparison to protein kinases an equally integral role in the control of cellular phospho-proteins. Concomitant control of kinases and phosphatases provides the cell with the capacity to rapidly switch proteins from their phosphorylated to dephosphorylated state to meet differing physiological demands.

Protein phosphatases are a diverse group of proteins that can be classified into two main families according to their substrate specificity: Serine/threonine phosphatases that dephosphorylate phospho-serine/-threonine residues and tyrosine phosphatases that dephosphorylate phospho-tyrosine residues. A third group, dual specificity protein phosphatases, which can dephosphorylate both phospho-serine/threonine and phospho-tyrosine residues, actually belongs to the tyrosine phosphatase family.

Molecular cloning has identified many protein serine/threonine phosphatases. PP1 (or type 1 phosphatase) and PP2A make up more than 90 % of the serine/threonine phosphatase activity in mammalian cells. PP1 phosphatases regulate a wide range of cellular processes, including cell-cycle progression, cell proliferation, protein synthesis, transcriptional regulation, and neurotransmission. PP1 is the major phosphatase that regulates glycogen metabolism in response to insulin and adrenalin. PP2A is supposed to function as a tumor suppressor.

To date, several dual specificity phosphatases have been identified in mamalian cells. As an example, CL100/3CH134 is shown to dephosphorylate threonine and tyrosine residues of extracellular signal-regulated kinases (ERK), thus leading to kinase inactivation. Since the initial cloning of CL100/3CH134, further dual specificity phosphatases have been identified. These include PAC1, hVH-2/MKP-2, hVH-3/B23 amongst others. These dual specificity phosphatases all appear to be effective in mediating inactivation of mitogen-activated protein (MAP) kinases (Camps, M. et al., The FASEB Journal, 14, 6 - 16, 2000).

Detecting the activity of protein phosphatases would be useful for the high-throughput screening of chemical libraries. Modulators of phosphatase activity could eventually be developed into drugs used for the treatment of e. g. Parkinson's disease, cancer, or diabetis mellitus and other metabolic disorders. A review of known protein phosphatase inhibitors is given by Oliver, C.J. and Shenolikar, S. (Frontiers in Bioscience 3, d961 - 972, September 1, 1998).

In a commercially available phosphatase assay, the release of phosphate from serine or threonine is detected by an absorbance change using Malachite Green as an indicator (Upstate Biotechnology, U.S.A., catalogue numbers # 14-110 and # 14-111). An assay principle based on the absorbency of the molybdate:malachit green:phosphate complex has also been disclosed in EP 0 874 052. The Malachit Green assay principle is a time consuming multi-step procedure and is highly sensitive to phosphate contaminations. Therefore, the different components of the phosphatase assay have to be carefully purified prior to the experiment.

In a further conventional approach, radioactivity is used to detect dephosphorylation of phospho-serine or phospho-threonine by the activity of a phosphatase by the release of ³²P from the substrate peptide or protein (Current Protocols in Molecular Biology, Unit 18.2, John Wiley & Sons). However, there is a growing demand away from the use of radioactivity in assay applications, because of problems with costs, safety, disposal, and shelf-life of the ligand reagents.

Unlike dephosphorylation of tyrosine, the development of preferably homogeneous assays to detect the dephosphorylation phospho-serine/-threonine by phosphatases, has been impeded to date by the lack of anti-phospho-serine/phospho-threonine antibodies available that bind specifically, and with high affinity, to phospho-serine or phospho-threonine residues.

It was therefore an object of the present invention to establish an assay method for detecting dephosphorylation of phospho-serine or phospho-threonine by phosphatases. The assay method is highly reliable and simple to perform without the need to develop specific high affinity anti-phospho-serine/phospho-threonine antibodies.

This object has been solved by the assay process accor-ding to the features of claim 1.

The present invention relates to a process for detecting dephosphorylation of phospho-serine/-threonine by phosphatase activity in an immunoassay which comprises the following steps:
a) providing a protein or peptide comprising the sequence motif

   -Z-X-Y- or -Y-X-Z-

   wherein
   Z = serine or threonine
   X = a sequence of between 1 and 10 amino acids which may be the same or different
   Y = tyrosine, serine or threonine
   as a substrate for the phosphatase, said protein or peptide being pre-phosphorylated at the Y and Z positions;
b) incubating the protein or peptide with the phosphatase to form a protein or peptide which is dephosphorylated at the Z position;
c) adding an antibody having a specificity to the peptide or protein that is phosphorylated in the Y and Z position; and
d) detecting the phosphatase activity.

By detecting the presence, absence or amount of a complex between bis-phosphorylated protein/peptide and antibody, a phosphatase activity can be measured.

The subclaims define preferred embodiments of the process of the present invention.

In the sequence motif, any amino acid or sequence of amino acids (X) can be inserted between Z and Y. The number of amino acids is in the range of 1 to 10 amino acids. A range of 1 to 10 is adequate to include both linear and conformational antibody epitopes. X is particularly at least one amino acid, any other short amino acid sequences having at least two amino acids, such as oligopeptides, being also included. For example, X is proline or glutamate or glycine.

The antibody used is usually a monoclonal or polyclonal antibody.

It has been shown that a particularly preferred antibody is a polyclonal antibody, for example a polyclonal antibody specific for activated JNK. It has been revealed that the antibody specifically recognizes a phosphorylated threonine or serine residue at the Z position within both a synthetic substrate peptide or activated JNK. Such antibodies are commercially available.

The immunoassay of the present invention may be performed as a direct binding immunoassay, preferably a homogeneous direct binding immunoassay.

A homogeneous direct binding immunoassay is based on a mix and measure principle. This is highly advantageous over the conventional direct binding assays always requiring complicated separation steps of the assay components before detection.

In the direct binding immunoassay, a labelled peptide or protein or a labelled antibody is used. The labelling may be carried out according to conventional standard techniques. Preferably, the peptide/protein or antibody is labelled using a luminescent or a radioactive tag or by using specific labelling molecules such as a reporter enzyme or an affinity ligand.

The assay of the present invention may be also be performed as a competition immunoassay, preferably a homogeneous indirect binding immunoassay. A homogeneous indirect binding immunoassay is also based on the mix and measure principle.

In this indirect binding immunoassay, a labelled bis-phosphorylated ligand is added to compete with the bis-phosphorylated peptide or protein for binding to the antibody. The ligand is preferably labelled using a luminescent or a radioactive tag or by using specific labelling molecules such as a reporter enzyme or an affinity ligand.

In case of using a protein containing the above motif, any protein containing the motif may be used. For example, a protein such as the JNK protein is used, which is the c-Jun N-terminal kinase, and which is also known in the literature as the stress-activated protein kinase 1 (SAPK1).

When it is more convenient to use a peptide substrate, the peptide sequence is preferably selected from the active-site loop, e.g. for PP1 or PP2A from the JNK1/2/3 active site. For instance, said peptide for PP1 or PP2A comprises or is composed of the amino acid sequence H-Lys-Phe-Met-Met-pThr-Pro-pTyr-Val-Val-Thr-Arg-NH₂, wherein p means phosphorylated.

When the incubation of the protein or peptide is carried out in the presence of a serine or threonine phosphatase, the phosphatase is preferably a serine/threonine protein phosphatase type 2A (PP2A) or type 2B (PP2B). In a further preferred embodiment, the incubation might be carried out using PP1, PP4 (also named PPX) or PP6 (also named PPV) as a phosphatase.

However, it is also possible to perform the assay utilising a tyrosine phosphatase with double specificity. Examples for such phosphatases comprise CL100/3CH134, PAC1, hVH-2/MKP-2, hVH-3/B23, hVH-5, MKP-3/PYST1, B59, MKP-4, and MKP-5.

The present invention also relates to a kit for detecting phosphatase activity in an immunoassay which comprises the following components:
- a phosphatase;
- a bis-phosphorylated substrate, i. e., a protein or peptide comprising the sequence motif

   Z-X-Y or -Y-X-Z

   wherein
   Z = serine or threonine
   X = a sequence of between 1 and 10 amino acids which may be the same or different
   Y = tyrosine, serine or threonine
   as a substrate for the phosphatase, said protein or peptide being pre-phosphorylated at the Y and Z positions;
- an antibody having a specificity to the peptide or protein that is phosphorylated in the Y and Z positon.

The kit may further comprise a detectable ligand, preferably a luminescently labelled ligand which comprises the sequence motif

-Z-X-Y- or -Y-X-Z-

wherein
Z = serine or threonine
X = a sequence of between 1 and 10 amino acids which may be the same or different
Y = tyrosine, serine or threonine
wherein said protein or peptide ligand is phosphorylated at the X and Z positions.

Among the phosphatases available, the phosphatase contained in the kit of the invention may be a serine or threonine phosphatase. It is also possible to use a dual specificity tyrosine phosphatase. Examples of the serine or threonine phosphatase as well as the dual specificity tyrosine phosphatase are described above.

The present invention also relates to a detectable ligand, preferably a luminescently labelled ligand, said ligand comprising the following sequence motif

-Z-X-Y- or -Y-X-Z-

wherein
Z = serine or threonine
X = a sequence of between 1 and 10 amino acids which may be the same or different
Y = tyrosine, serine or threonine
Wherein said protein or peptide ligand is phosphorylated at the Y and Z positions.

The process of the present invention as well as the kit and the labelled ligand may be used for screening for specific modulators of serine or threonine or dual specificity phosphatase activity. The assay is particularly suitable for screening compound libraries in order to locate molecules which inhibit or activate phosphatases. These molecules may be promising candidates for designing drugs used for the treatment of e.g. metabolic disorders and cancer.

The accompanying figures illustrate the present invention. Abbreviations for the described peptides are used as follows:

P1°        H-Lys-Phe-Met-Met-Thr-Pro-pTyr-Val-Val-Thr-Arg-NH2
P1*        H-Lys-Phe-Met-Met-pThr-Pro-Tyr-Val-Val-Thr-Arg-NH2
P1*°       H-Lys-Phe-Met-Met-pThr-Pro-pTyr-Val-Val-Thr-Arg-
           NH2

TAMRA-P1*°      5-TAMRA-AEEA-Lys-Phe-Met-Met-pThr-Pro-pTyr-
                Val-Val-Thr-Arg-NH2
TAMRA-P1°       5-TAMRA-AEEA-Lys-Phe-Met-Met-Thr-Pro-pTyr-Val-
                Val-Thr-Arg-NH2
TAMRA      5'-(6-carboxytetramethylrhodamine)
AEEA       8-amino-3,6-dioxaoctanoic acid linker

In the figures the following is shown:

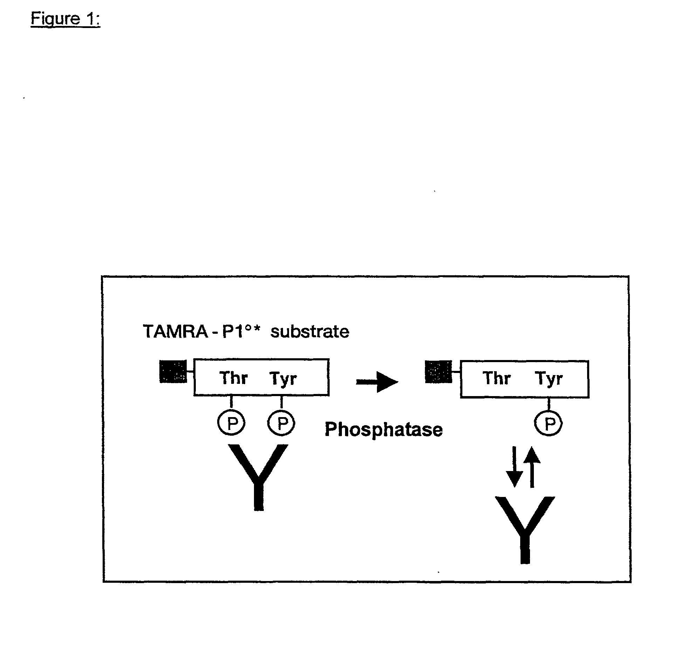

- Figure 1: is a schematic drawing of a preferred embodiment of the principle of the direct phosphatase assay of the present invention. The TAMRA-P1°* substrate having the amino acid sequence 5-TAMRA-AEEA-Lys-Phe-Met-Met-pThr-Pro-pTyr-Val-Val-Thr-Arg-NH₂ (p means phosporylated) becomes dephosphorylated at the threonine residue in the presence of the phosphatase. Upon dephosphorylation, the TAMRA-labelled P1°*-peptide will no more bind to the polyclonal anti-active JNK antibody.

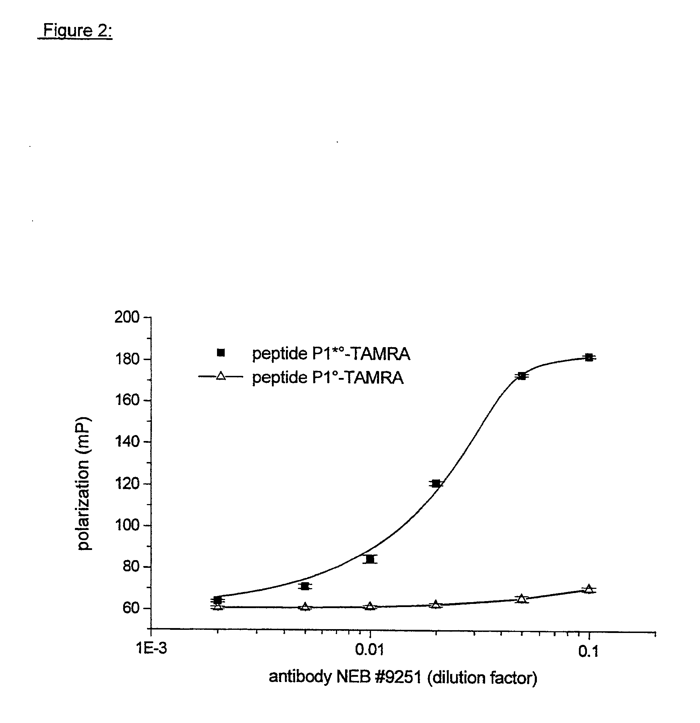

- Figure 2: is a diagram showing the specific binding of the polyclonal anti-active JNK antibody to TAMRA-labelled P1°*-peptide; as a control, no binding is measured for TAMRA-labelled P1°-peptide.

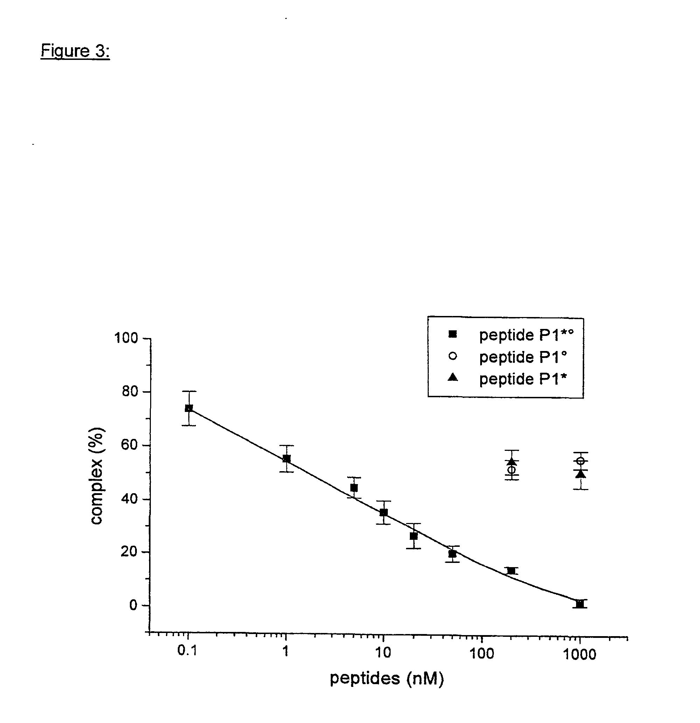

- Figure 3: is a diagram showing the competition of the binding of the polyclonal JNK antibody to the TAMRA-labelled P1°*-peptide using double-phosphorylated P1°*-peptide (determination of the IC50 for the P1°*-peptide competitor) and, as controls, mono-phosphorylated P1°- and P1*-peptides.

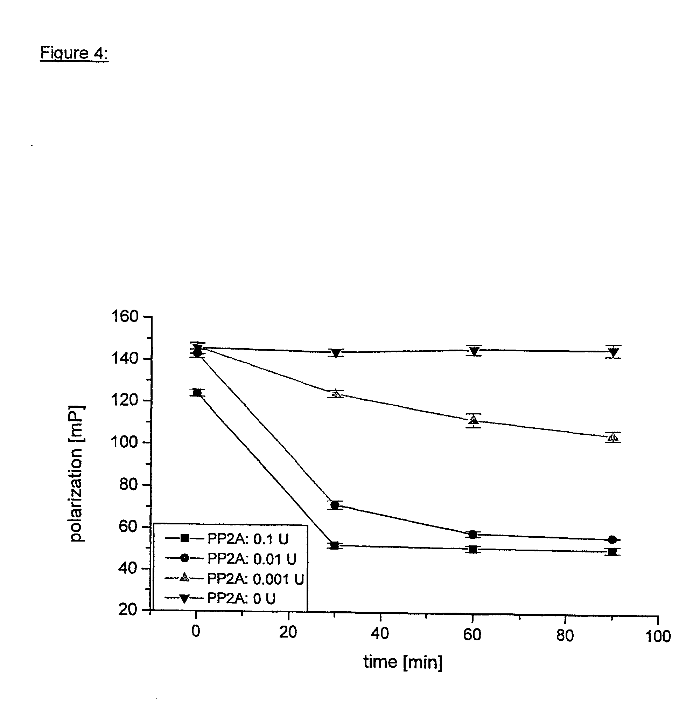

- Figure 4: is a diagram of a direct phosphatase assay (see Fig. 1) showing a time course of the dephosphorylation of the TAMRA-P1°*-peptide at different PP2A phosphatase concentrations. Desphosphorylated TAMRA-P1°*-peptide does not bind to the polyclonal anti-active JNK antibody, resulting in low fluorescence polarization.

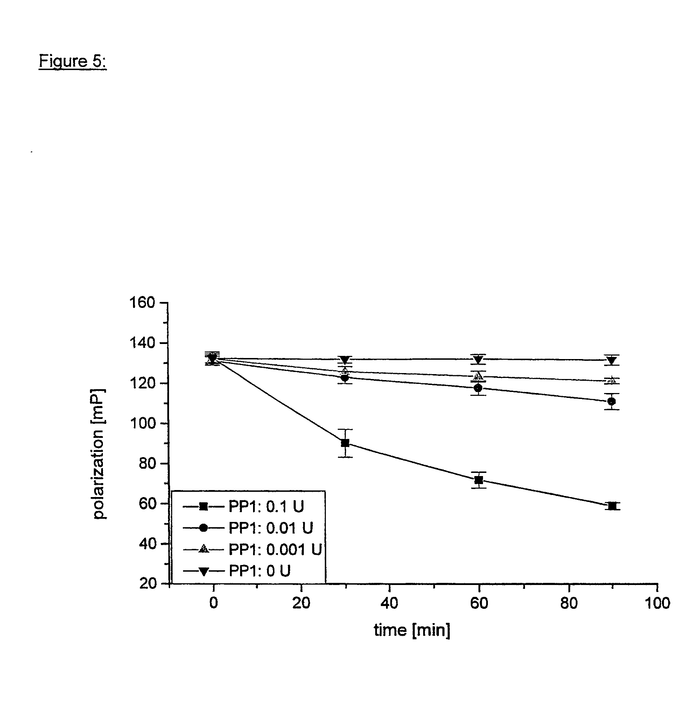

- Figure 5: is a diagram of a direct phosphatase assay (see Fig. 1) showing a time course of the dephosphorylation of the TAMRA-P1°*-peptide at different PP1 phosphatase concentrations. Desphosphorylated TAMRA-P1°*-peptide does not bind to the polyclonal anti-active JNK antibody, resulting in low fluorescence polarization.

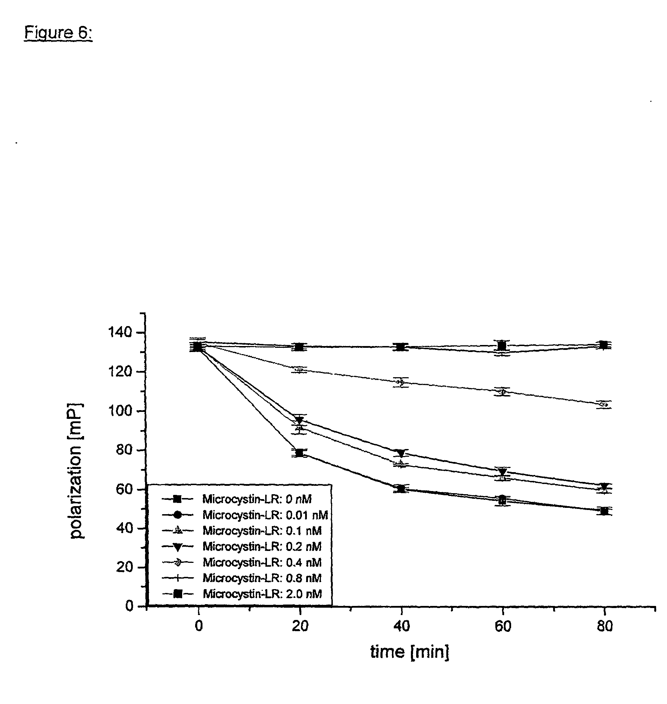

- Figure 6: is a diagram of a direct phosphatase assay (see Fig. 1) showing the inhibition of the dephosphorylation of the TAMRA-P1°*-peptide by PP2A phosphatase at different microcystin-LR (phosphatase inhibitor) concentrations. Desphosphorylated TAMRA-P1°*-peptide does not bind to the polyclonal anti-active JNK antibody, resulting in low fluorescence polarization.

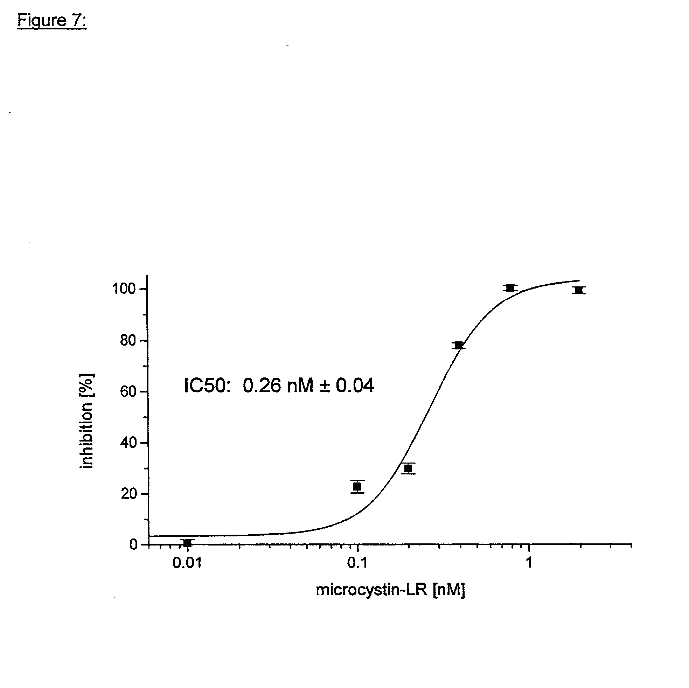

- Figure 7: is a diagram showing the inhibition of the PP2A-dependent dephosphorylation of the TAMRA-P1°*-peptide substrate by the phosphatase inhibitor microcystin-LR. From the inhibition curve, the half-maximal inhibition concentration (IC50) is calculated.

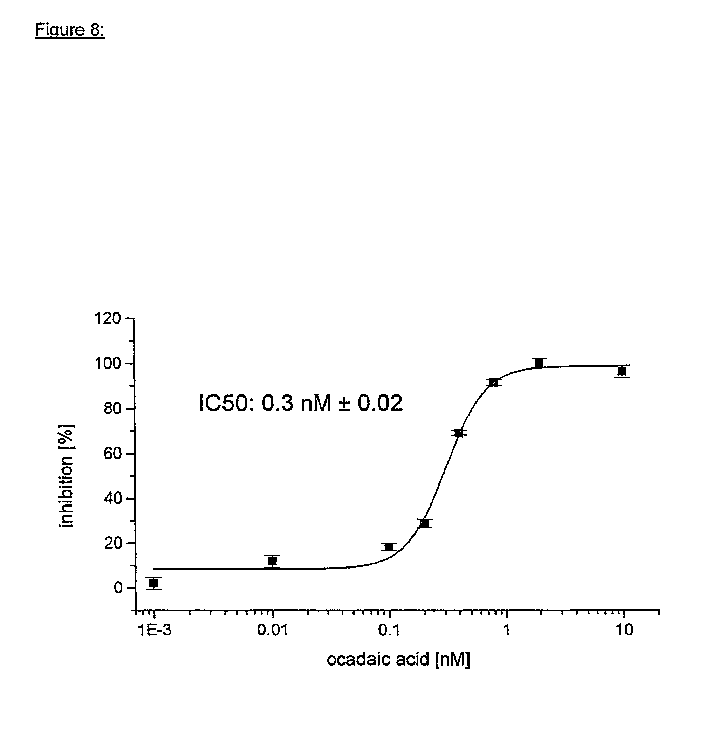

- Figure 8: is a diagram showing the inhibition of the PP2A-dependent dephosphorylation of the TAMRA-P1°*-peptide substrate by the phosphatase inhibitor ocadaic acid. From the inhibition curve, the half-maximal inhibitory concentration (IC50) is calculated.

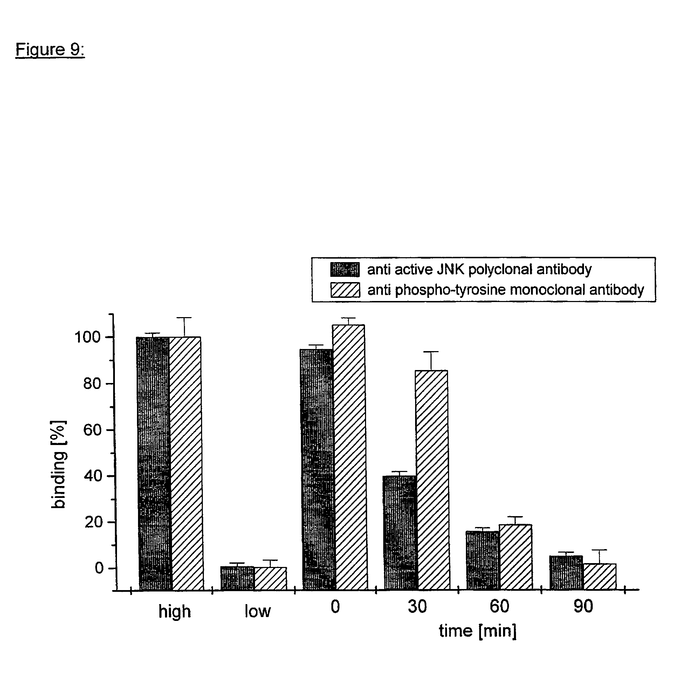

- Figure 9: is a diagram of a direct phosphatase assay (see Fig. 1) showing a time course of the dephosphorylation of the TAMRA-P1°*-peptide by PP1 phosphatase which is detected by two different antibodies. The polyclonal anti-active JNK antibody detects both the desphosphorylation of the phospho-threonine and/or the dephosphorylation of the phospho-tyrosine. As a control, the monoclonal anti-phospho-tyrosine antibody only detects the dephosphorylation of the phospho-tyrosine. Both events result in low fluorescence polarization. The controls denoted as "high" mean maximum binding of the antibodies to the TAMRA-P1°* peptide. The controls denoted as "low" mean no binding of the antibodies to the TAMRA-P1°* peptide due to addition of 200 nM P1°* competitor peptide.

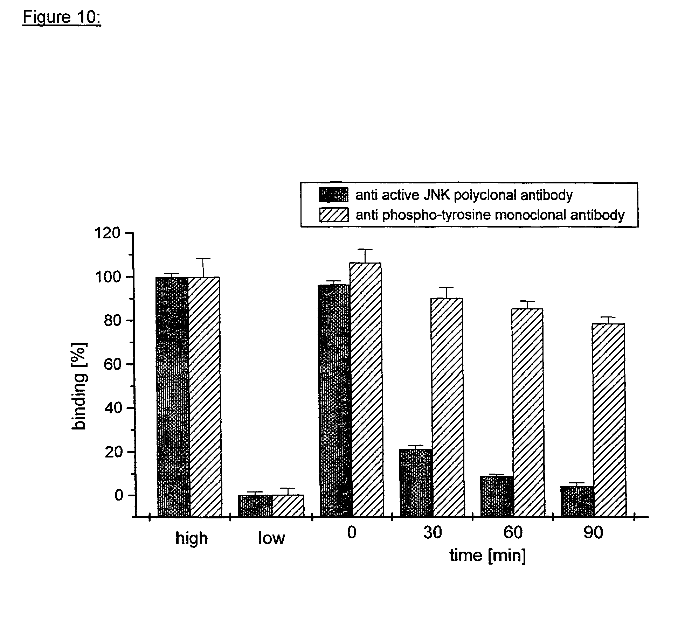

- Figure 10: is a diagram of a direct phosphatase assay (see Fig. 1) showing a time course of the dephosphorylation of the TAMRA-P1°*-peptide by PP2A phosphatase which is detected by two different antibodies. The polyclonal anti-active JNK antibody detects both the desphosphorylation of the phospho-threonine and/or the dephosphorylation of the phospho-tyrosine. The monoclonal anti-phospho tyrosine antibody only detects the dephosphorylation of the phospho-tyrosine. Both events result in low fluorescence polarization. The controls denoted as "high" mean maximum binding of the antibodies to the TAMRA-P1°* peptide. The controls denoted as "low" mean no binding of the antibodies to the TAMRA-P1°* peptide due to addition of 200 nM P1°* competitor peptide.

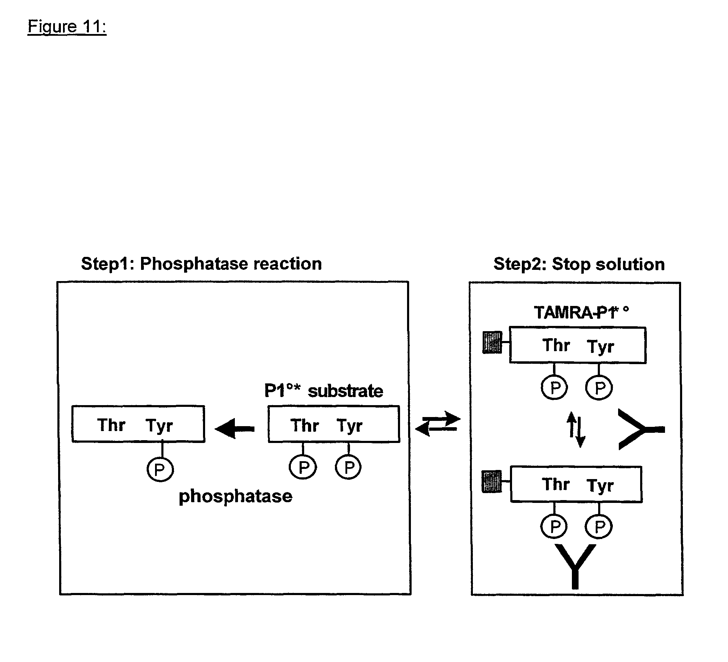

- Figure 11: is a schematic drawing of the principle of the indirect phosphatase assay of the present invention. In a first step, the bis-phosphorylated non-fluorescent P1°*-peptide is dephosphorylated by the activity of a phosphatase. The product of the reaction (dephosphorylated P1°* peptide) will no more compete with the TAMRA-labelled P1°*-peptide for the binding to the polyclonal anti-active JNK antibody which are both added in a second step (stop solution including a reagent inactivating the phosphatase).

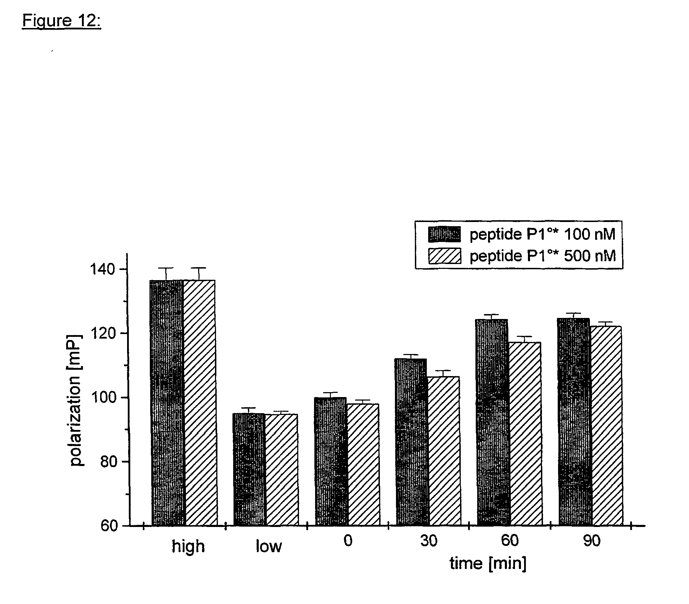

- Figure 12: is a diagram of an indirect phosphatase assay (see Fig. 11) showing a time course of the dephosphorylation of the P1°*-peptide substrate by PP2A phosphatase at different P1°*-peptide concentrations. Desphosphorylated P1°*-peptide does no more compete with the TAMRA-labelled P1°*-peptide for the binding to the polyclonal anti-active JNK antibody, resulting in high fluorescence polarization. The controls denoted as "high" mean maximum binding of the antibodies to the TAMRA-P1°* peptide. The controls denoted as "low" mean no binding of the antibodies to the TAMRA-P1°* peptide due to the presence of 100 nM or 500 nM P1°* substrate peptide.

The following experiments further illustrate the present invention.

### Example 1:

### Measurement of the Binding Affinity (KD) of the anti-active JNK Antibody (New England Biolabs NEB #9251)

This polyclonal anti-active antibody detects all three isoforms of the SAPK1/JNK proteins only when they are activated by dual phosphorylation at threonine₁₈₃/tyrosine₁₈₅. Binding of polyclonal anti-active JNK antibody #9251 (concentration: 0.02 mg/ml, equiv. 130 nM) to TAMRA-labelled P1*° peptide (at 5 nM) was measured at different antibody concentrations by fluorescence polarization. As a control, no binding of the antibody to the monophosphorylated TAMRA-P1° peptide (at 5 nM) is detected.

Affinity of the polyclonal anti active JNK antibody for peptide P1*°-TAMRA: K_{D} = 2.6 nM

The results of this experiment are illustrated in Fig. 2. It has been revealed that the commercially available polyclonal anti-active JNK-specific antibodies are particularly useful in the assay of the present invention. As can be seen in Fig. 2, the polyclonal anti-active JNK antibody detects the peptides - derived of the active site loop of JNK protein - only when dual phosphorylated at threonine₁₈₃ and tyrosine₁₈₅. The polarization values dramatically increase when using the double-phosphorylated TAMRA-P1*° peptide in contrast to mono- phosphorylated labelled TAMRA-P1° peptide (sequences see above).

### Example 2:

### Determination of IC50 for Peptide P1°*:

Competition of NEB #9251 antibody-P1°*-TAMRA complex with P1°* peptide was measured by fluorescence correlation spectroscopy: Binding of anti-active JNK antibody NEB #9251 (1:20 diluted) to TAMRA-labelled peptide P1*° (5 nM) was competed with non-fluorescent, double-phosphorylated peptide P1*°. Control peptides: mono-phosphorylated P1° and P1* did not compete effectively. Peptide P1*°: IC₅₀ = 5.0 nM ± 3.3 nM

The results of this experiment are illustrated in Fig.3. In Fig. 3 the binding of the same polyclonal anti-active antibody to TAMRA-labelled peptide P1*°(5 nM) in competition with the peptides P1° (mono-phosphorylated, sequence see above), peptide P1* (mono-phosphorylated, sequence see above) or peptide P1*° (double phosphorylated, sequence see above) is presented. As can be deduced from the complex formation, only bis-phosphorylated P1*° peptide is able to compete effectively with the TAMRA-labelled P1*°(peptide ligand) for binding to the antibody while the other mono-phosphorylated peptides displace less than 50% of the bound ligand even at high concentrations (up to 1 µM).

### Example 3:

### Dephosphorylation of TAMRA-P1°* Peptide by PP2A Phosphatase:

In Fig. 4 the rate of dephosphorylation of the P1°*-TAMRA substrate peptide by PP2A phosphatase is determined at various PP2A concentrations. Efficient dephosphorylation of the P1°*-TAMRA substrate peptide using the direct phosphatase assay is performed as follows: In a total volume of 80 µl the P1°*-TAMRA substrate peptide (at 10 nM) is dephosphorylated using 0.1, 0.01, or 0,001 units of PP2A. The phosphatase reactions are incubated at 30°C and aliquots (20 µl) are withdrawn after 0 min, 30 min, 60 min and 90 min and mixed with 10 µl of a stop solution containing the following reagents (all final concentrations): hydrogenperoxide 10 mM, polyclonal anti-active JNK antibody from NEB at a dilution of 1:20.

The formed dephosphorylated P1°*-TAMRA peptide product can be detected as it does not bind to the polyclonal anti-active JNK antibody. As a results, a drop of the fluorescence polarization is detected which is inversely proportional to PP2A phosphatase activity.

### Example 4:

### Dephosphorylation of TAMRA-P1°* Peptide by PP1 Phosphatase:

In Fig. 5 the rate of dephosphorylation of the P1°*-TAMRA substrate peptide by PP1 phosphatase is detected at various PP1 concentrations using the same direct assay as described in example 3. Efficient dephosphorylation of the P1°*-TAMRA substrate peptide using the direct phosphatase assay is performed as follows: In a total volume of 80 µl the P1°*-TAMRA substrate peptide (at 10 nM) is dephosphorylated using 0.1, 0.01, or 0,001 units of PP1. The phosphatase reactions are incubated at 30°C and aliquots (20 µl) are withdrawn after 0 min, 30 min, 60 min and 90 min and mixed with 10 µl of a stop solution containing the following reagents (all final concentrations): hydrogenperoxide 10 mM, polyclonal anti-active JNK antibody from NEB (1:20 dilution). The formed dephosphorylated P1°*-TAMRA peptide product can be detected as it does not bind to the polyclonal anti-active JNK antibody. As a results, a drop of the fluorescence polarization is detected which is inversely proportional to PP1 phosphatase activity.

### Example 5:

### Inhibition of the PP2A Phosphatase-dependent Dephosphorylation of TAMRA-P1°* Peptide by Microcystin-LR:

In Fig. 6 the inhibition of the dephosphorylation of P1°*-TAMRA substrate peptide by PP2A phosphatase is determined for the phosphatase inhibitor microcystin-LR. The inhibition of PP2A phosphatase is detected using the same direct assay as described in example 3. As a result, no reduction of the binding of the TAMRA-labelled P1*° peptide to the polyclonal anti-active JNK antibody is detected at high microcystin-LR concentrations due to full inhibition of PP2A.

Assay conditions: PP2A phosphatase (0.004 units) is incubated with P1°*-TAMRA peptide (10 nM) and microcystin-LR (0, 0.01, 0.1, 0.2, 0.4, 0.8, 2 nM) in a total volume of 100 µl at 30°C. At different time points aliquots are withdrawn from the enzyme reaction and mixed with hydrogen peroxide (10 nM), polyclonal anti-active JNK antibody from NEB #9251 (1:20 dilution).

### Example 6:

### Inhibition of the PP2A Phosphatase-dependent Dephosphorylation of TAMRA-P1°* Peptide by Microcystin-LR:

Fig. 7 shows the inhibition of the dephosphorylation of P1°*-TAMRA substrate peptide by PP2A phosphatase using the phosphatase inhibitor microcystin-LR. The half-maximal inhibitory concentration (IC50) for microcystin-LR is calculated from the data of Fig. 6: IC50 = 0.26 nM +/-0.04 nM.

### Example 7:

### Inhibition of the PP2A Phosphatase-dependent Dephosphorylation of TAMRA-P1°* Peptide by Ocadaic Acid:

Fig. 8 shows the inhibition of the dephosphorylation of P1°*-TAMRA substrate peptide by PP2A phosphatase using the phosphatase inhibitor ocadaic acid. The half-maximal inhibitory concentration (IC50) for ocadaic is calculated. IC50 = 0.3 nM +/- 0.02 nM.

Assay conditions: PP2A phosphatase (0.003 units) is incubated with P1°*-TAMRA peptide (10 nM) and ocadaic acid (0, 0.001, 0.01, 0.1, 0.2, 0.4, 0.8, 2, 10 nM) at 30°C. At different time points aliquots are withdrawn from the enzyme reaction and mixed with hydrogen peroxide (10 nM), polyclonal anti-active JNK antibody from NEB #9251 (1:20 dilution).

### Example 8:

### Measurement of the Specificity of PP1 phosphatase using two different Antibodies:

Fig. 9 shows the detection of PP1 phosphatase-dependent dephosphorylation of TAMRA-P1°* Peptide using the polyclonal anti-active JNK antibody (NEB #9251) and the monoclonal anti phospho-tyrosine antibody (NEB #9411). The dephosphorylation of the TAMRA-P1°* peptide by PP1 phosphatase is performed using the same direct assay as described in Fig 5, with the only exception that two antibodies with different specificities were used for the read-out.

As a result, a PP1 phosphatase-dependent dephosphorylation of the phospho-threonine and the phospho-tyrosine is detected. PP1 has a lower specificity when compared to PP2A (see example 9).

Assay conditions: PP1 phosphatase (0.1 units) is incubated with P1°*-TAMRA peptide (10 nM) at 30°C. At different time points aliquots are withdrawn from the enzyme reaction and mixed with the stop solution containing hydrogen peroxide (10 nM) along with either the polyclonal anti-active JNK antibody from NEB #9251 (1:20 dilution) or the monoclonal anti phospho-tyrosine antibody from NEB #9411 (1:100 dilution)

### Example 9:

### Measurement of the Specificity of PP2A phosphatase using two different Antibodies:

Fig. 10 shows the detection of PP2A phosphatase-dependent dephosphorylation of TAMRA-P1°* Peptide using the polyclonal anti active JNK antibody NEB #9251 and the monoclonal anti phospho-tyrosine antibody NEB #9411. The dephosphorylation of the TAMRA-P1°* peptide by PP2A phosphatase is performed using the same direct assay as described in Fig 5, with the only exception that two antibodies with different specificities were used for the read-out. As a result, a PP2A phosphatase-dependent dephosphorylation of the phospho-threonine is detected. PP2A has a high specificity for phospho-threonine.

Assay conditions: PP2A phosphatase (0.006 units) is incubated with P1°*-TAMRA peptide (10 nM) at 30°C. At different time points aliquots are withdrawn from the enzyme reaction and mixed with the stop solution containing hydrogen peroxide (10 nM) along with either the polyclonal anti active JNK antibody from NEB #9251 (1:20 dilution) or the monoclonal anti phospho-tyrosine antibody from NEB #9411 (1:100 dilution)

### Example 10:

### Dephosphorylation of non-fluorescent P1°* Peptide by PP2A Phosphatase (indirect Phosphatase Assay, see Fig. 11):

Fig. 12 shows the detection of PP2A phosphatase-dependent dephosphorylation of the P1°* substrate peptide in an indirect phosphatase assay.

In a first step (enzyme reaction), the bisphosphorylated P1°* substrate peptide is incubated with PP2A. In a second step (stop solution, including hydrogenperoxide), the polyclonal anti active JNK antibody (NEB #9251) is added together with TAMRA-P1°* peptide. As a result of the PP2A activity, the P1°* substrate peptide is dephosphorylated and does no longer compete with the TAMRA-P1°* peptide for the binding to the polyclonal anti active JNK antibody. This can be detected by an increase in fluorescence polarization.

Assay conditions: PP2A phosphatase (0.5 units) is incubated with 100 nM or 500 nM P1°* substrate peptide in a total volume of 80 µl at 30°C. At different time points, aliquots (20 µl) are withdrawn from the enzyme reaction and mixed with 10 µl of the stop solution containing hydrogen peroxide (10 nM), TAMRA-P1°* peptide at (5 nM) together with the polyclonal anti active JNK antibody from NEB (1:20 dilution).

### List of reagents of the described phosphatase assays:

### List of used peptides:

TAMRA-P1*° 5-TAMRA-AEEA-Lys-Phe-Met-Met-pThr-Pro-pTyr-
Val-Val-Thr-Arg-NH2
TAMRA-P1° 5-TAMRA-AEEA-Lys-Phe-Met-Met-Thr-Pro-pTyr-Val-
Val-Thr-Arg-NH2
P1*° H-Lys-Phe-Met-Met-pThr-Pro-pTyr-Val-Val-Thr-Arg-
NH2
P1° H-Lys-Phe-Met-Met-Thr-Pro-pTyr-Val-Val-Thr-Arg-NH2
P1* H-Lys-Phe-Met-Met-pThr-Pro-Tyr-Val-Val-Thr-Arg-NH2

### Bisphosphorylated substrate peptide:

TAMRA-P1*°-Peptide 5-TAMRA-AEEA-Lys-Phe-Met-Met-pThr-Pro-pTyr-
Val-Val-Thr-Arg-NH2
Supplier: EVOTEC inhouse solid-phase peptide synthesis
HK-03-65-P1-13; M = 2089 g/mol; MALDI (2092.18)
1mM stock solution in 100% DMSO

### Bisphosphorylated competitor peptide:

P1*°-Peptide: H-Lys-Phe-Met-Met-pThr-Pro-pTyr-Val-Val-Thr-
Arg-NH2
Supplier: EVOTEC inhouse solid-phase peptide synthesis
HK-03-60-P1-7; M = 1532 g/mol; MALDI (1531.88)
10mM stock solution in 100% DMSO

### Monophosphorylated control peptides:

P1°-Peptide: H-Lys-Phe-Met-Met-Thr-Pro-pTyr-Val-Val-Thr-
Arg-NH2
Supplier: EVOTEC in house solid-phase peptide synthesis
HK-03-58-HF; M = 1451 g/mol; MALDI (1453.57)
10mM stock solution in 100% DMSO

P1*-Peptide:  H-Lys-Phe-Met-Met-pThr-Pro-Tyr-Val-Val-Thr-
Arg-NH2
Supplier: EVOTEC in house solid-phase peptide synthesis
HK-03-33-HF; M = 1452 g/mol; MALDI (1452.23)
10mM stock solution in 100% DMSO

### Enzyme:

### PP1 phosphatase:

Supplier: Upstate Biotech cat. no. 14-110, lot no. 18524

### PP2A phosphatase:

Supplier: Upstate Biotech cat. no. 14-111, lot no. 20574.

### Anti active JNK-specific antibody:

New England Biolabs, U.S.A.: NEB #9251

### Anti phospho-tyrosine-specific monoclonal antibody pY100:

New England Biolabs, U.S.A.: NEB #9411

### Reagents and buffers:

### Assay buffer:

*10x HEPES-Assay-Buffer pH 7.2*
*500mM HEPES*
*100mM MgCl*_{*2*}
*10mM DTT*

Dissolve 77mg DTT (FLUKA cat. no. 43815; MW 154.25g/mol) [final concentration 10mM] in 30 ml of Millipore water, add 5ml of 1M MgCl₂ [final concentration 100mM], and 5.96g HEPES (FLUKA cat. no. 54457) [final concentration 500mM] and adjust pH with 1M NaOH to pH 7.2. Top up to 50 ml with Millipore water. The buffer will be filtered sterile (0.22µm) aliquoted to 1.5ml and should be stored at -20°C.

### 1M MgCl₂:

Dissolve 10.2 g MgCl₂.6H₂O (FLUKA cat. no. 63068) in 50 ml of Millipore water. Buffer should be filtered (0.42µm) and can be stored at 4°C for several months.

### 1% (w/v) Pluronic F-127

Dissolve 0.5 g Pluronic (Sigma, cat. no. P-2443) in 50 ml of Millipore water. Stir gently to get a clear solution. Solution should be filtered (0.42µm) and can be stored at 4°C for several months.

### 1x HEPES-Assay-Buffer/0.1% Pluronic/ 0.01% BSA pH 7.5: (2ml) :

*50mM HEPES*
*10mM MgCl*_{*2*}
*1mM DTT*
*0.1% Pluronic*
*0.01% BSA*

Add 50 µl 4% (w/v) Pluronic F-127 in water, add 200 µl of 10x HEPES-Assay-Buffer pH 7.5 and add 20 µl of 1% BSA in water (Merck Albumin Fraktion V) to 2 ml of Millipore water.

### 1%(w/v) Albumin Fraktion V (BSA)

Dissolve 0.1g Albumin Fraktion V (Merck cat.no.1.12018.0100) in 10ml of steril filtered (0.22µm) Millipore water and aliquote to 100µl. The stock should be stored at -20°C.

### DMSO: 100% DMSO

The solvent was purchased from SIGMA (cat. No. P-2650), sterile filtered.

### Hydrogenperoxide 30% (H₂O₂)

*Add 10µl of 30%* H₂O₂ (Merck cat.no.8.22287.1000) in 78 µl 1x HEPES-Assay-Buffer/0.1% Pluronic/ 0.01% BSA pH 7.5 (final concentration 1M).

### Microcystin-LR:

Supplier: Sigma cat.no. M-2912, lot no. 48H1100

### Ocadaic Acid:

Supplier: Sigma cat.no. 0-7760, lot no. 129H1199

## Claims

1. A process for detecting phosphatase activity in an immunoassay comprising the following steps:
a) providing a protein or peptide comprising the sequence motif
-Z-X-Y- or -Y-X-Z-
wherein
Z = serine or threonine
X = a sequence of between 1 and 10 amino acids which may be the same or different
Y = tyrosine, serine or threonine
as a substrate for a phosphatase, said protein or peptide being pre-phosphorylated at the Y and Z positions;
b) incubating the protein or peptide with a phosphatase to form a protein or peptide which is dephosphorylated at the Z position;
c) adding an antibody having a specificity to the peptide or protein that is phosphorylated in the Y and Z position; and
d) detecting the phosphatase activity.

2. The process according to claim 1, wherein the immunoassay is performed as a direct binding immunoassay, preferably a homogeneous direct binding immunoassay.

3. The process according to claim 2, wherein a labelled peptide or protein is used.

4. The process according to claim 2, wherein a labelled antibody is used.

5. The process according to claim 3 or 4, wherein the peptide/protein or antibody is labelled by a luminescent tag, a radioactive marker, a reporter enzyme or an affinity ligand.

6. The process according to claim 1, wherein the immunoassay is performed as an indirect binding immunoassay, preferably a homogeneous indirect binding immunoassay.

7. The process according to claim 6, wherein a labelled bis-phosphorylated ligand is added to compete with the bis-phosphorylated protein or peptide for binding to the antibody, said ligand being phosphorylated at the Y and Z positions.

8. The process according to claim 7, wherein the ligand being bis-phosphorylated at the Y and Z positions is labelled by a luminescent tag, a radioactive marker, a reporter enzyme or an affinity ligand.

9. The process according to claim 8 wherein the ligand is 5-Tamra-AEEA-Lys-Phe-Met-Met-pThr-Pro-pTyr-Val-Val-Thr-Arg-NH₂, with AEEA denoting an 8-amino-3,6-dioxaoctanoic acid linker.

10. The process according to at least one of claims 1 to 9, wherein the assay is performed as a fluorescence immunoassay, in particular a fluorescence polarization immunoassay, a fluorescence correlation spectroscopic assay, a fluorescence lifetime assay, or a fluorescence intensity distribution assay.

11. The process according to at least one of claims 1 to 10, wherein X in the sequence motif comprises proline or glutamate or glycine.

12. The process according to at least one of claims 1 to 11, wherein the protein provided is activated JNK1, JNK2, or JNK3 protein.

13. The process according to at least one of claims 1 to 11, wherein the peptide provided includes sequences identical to those of the activated JNK1, JNK2 or JNK3 active-site loop.

14. The process according to claims 1 to 11 wherein the peptide comprises the amino acid sequence H-Lys-Phe-Met-Met-pThr-Pro-pTyr-Val-Val-Thr-Arg-NH₂ (p means phosphorylated).

15. The process according to claim 1 to 11 wherein the peptide is composed of the amino acid sequence H-Lys-Phe-Met-Met-pThr-Pro-pTyr-Val-Val-Thr-Arg-NH₂ (p means phosphorylated).

16. The process according to at least one of claims 1 to 15 wherein the phosphatase is a serine or threonine phosphatase.

17. The process according to claim 16, wherein the incubation of the protein or peptide is carried out in the presence of a serine/threonine protein phosphatase type 2A(PP2A) or type 2B (PP2B).

18. The process according to claim 16, wherein the incubation of the protein or peptide is carried out in the presence of the phosphatase PP1.

19. The process according to claim 16, wherein the incubation of the protein or peptide is carried out in the presence of the phosphatase PP4 (also named PPX) or PP6 (also named PPV).

20. The process according to at least one of claims 1 to 15 wherein the phosphatase is a tyrosin phosphatase with dual specificity.

21. The process according to claim 20 wherein the dual specificity phosphatase is CL100/3CH134, or PAC1, or hVH-2/MKP-2, or hVH-3/B23, or hVH-5, or MKP-3/PYST1, or B59, or MKP-4, or MKP-5.

22. The process according to at least one of claims 1 to 21 wherein the protein or peptide is dephosphorylated at the Y position by the action of the phosphatase.

23. The process according to at least one of claims 1 to 22, wherein the antibody is a monoclonal or polyclonal antibody.

24. The process according to claim 23 wherein the antibody is a polyclonal antibody.

25. The process according to claim 23 wherein the antibody is a polyclonal antibody specific for active JNK.

26. A kit for detecting phosphatase activity in an immunoassay comprising the following components:
- a phosphatase;
- a substrate as defined in claim 1;
- an antibody as defined in claim 1.

27. The kit according to claim 26 wherein the phosphatase is a serine or threonine phosphatase.

28. The kit according to claim 26 wherein the phosphatase is a dual specificity tyrosine phosphatase.

29. Use of the assay process according to at least one of the claims 1 to 25 or the kit according to at least one of the claims 26 to 28 for screening modulators for phosphatase activity, in particular inhibitors for a serine or threonine or dual specificity phosphatase.

## Patentansprüche

1. Verfahren zum Nachweis von Phosphatase-Aktivität in einem Immunoassay, umfassend die folgenden Schritte:
a) Bereitstellung eines Proteins oder Peptids, umfassend das Sequenzmotiv
-Z-X-Y- oder -Y-X-Z-
worin
Z = Serin oder Threonin
X = eine Sequenz von zwischen 1 und 10 Aminosäuren, welche gleich oder verschieden sein können,
Y = Tyrosin, Serin oder Threonin,
als Substrat für eine Phosphatase, wobei das Protein oder Peptid an den Y- und Z-Positionen vorphosphoryliert ist;
b) Inkubation des Proteins oder Peptids mit einer Phosphatase zur Bildung eines Proteins oder Peptids, welches an der Z-Position dephosphoryliert ist;
c) Zugabe eines Antikörpers mit einer Spezifität für das Peptid oder Protein, das an der Y- und Z-Position phosphoryliert ist; und
d) Nachweis der Phosphatase-Aktivität.

2. Verfahren nach Anspruch 1, wobei der Immunoassay als ein Immunoassay mit direkter Bindung, vorzugsweise ein homogener Immunoassay mit direkter Bindung, durchgeführt wird.

3. Verfahren nach Anspruch 2, wobei ein markiertes Peptid oder Protein verwendet wird.

4. Verfahren nach Anspruch 2, wobei ein markierter Antikörper verwendet wird.

5. Verfahren nach Anspruch 3 oder 4, wobei das Peptid/Protein oder der Antikörper mit einer Lumineszenzmarkierung, einem radioaktiven Marker, einem Reporterenzym oder einem Affinitätsliganden markiert ist.

6. Verfahren nach Anspruch 1, wobei der Immunoassay als ein Immunoassay mit indirekter Bindung, vorzugsweise ein homogener Immunoassay mit indirekter Bindung, durchgeführt wird.

7. Verfahren nach Anspruch 6, wobei ein markierter bisphosphorylierter Ligand zugegeben wird, um mit dem bisphosphorylierten Protein oder Peptid um die Bindung an den Antikörper zu konkurrieren, wobei der Ligand an den Y- und Z-Positionen phosphoryliert ist.

8. Verfahren nach Anspruch 7, wobei der Ligand, der an den Y- und Z-Positionen bisphosphoryliert ist, mit einer Lumineszenzmarkierung, einem radioaktiven Marker, einem Reporterenzym oder einem Affinitätsliganden markiert ist.

9. Verfahren nach Anspruch 8, wobei der Ligand 5-Tamra-AEEA-Lys-Phe-Met-Met-pThr-Pro-pTyr-Val-Val-Thr-Arg-NH₂ ist, wobei AEEA einen 8-Amino-3,6-dioxaoctansäure-Linker bezeichnet.

10. Verfahren nach mindestens einem der Ansprüche 1 bis 9, wobei der Assay als Fluoreszenzimmunoassay, insbesondere als ein Fluoreszenzpolarisationsimmunoassay, ein Fluoreszenzkorrelationsspektroskopie-Assay, ein Fluoreszenzlebensdauer-Assay oder ein Fluoreszensintensitätsverteilungs-Assay durchgeführt wird.

11. Verfahren nach mindestens einem der Ansprüche 1 bis 10, wobei X in dem Sequenzmotiv Prolin oder Glutamat oder Glycin umfaßt.

12. Verfahren nach mindestens einem der Ansprüche 1 bis 11, wobei das bereitgestellte Protein aktiviertes JNK1-, JNK2- oder JNK3-Protein ist.

13. Verfahren nach mindestens einem der Ansprüche 1 bis 11, wobei das bereitgestellte Peptid Sequenzen einschließt, die mit denjenigen der Schleife der aktiven Stelle von aktiviertem JNK1, JNK2 oder JNK3 identisch sind.

14. Verfahren nach Anspruch 1 bis 11, wobei das Peptid die Aminosäuresequenz H-Lys-Phe-Met-Met-pThr-Pro-pTyr-Val-Val-Thr-Arg-NH₂ (p bedeutet phosphoryliert) umfaßt.

15. Verfahren nach Anspruch 1 bis 11, wobei das Peptid aus der Aminosäuresequenz H-Lys-Phe-Met-Met-pThr-Pro-Tyr-Val-Val-Thr-Arg-NH₂ (p bedeutet phosphoryliert) besteht.

16. Verfahren nach mindestens einem der Ansprüche 1 bis 15, wobei die Phosphatase eine Serin- oder Threoninphosphatase ist.

17. Verfahren nach Anspruch 16, wobei die Inkubation des Proteins oder Peptids in Gegenwart einer Serin/Threonin-Proteinphosphatase vom Typ 2A (PP2A) oder Typ 2B (PP2B) durchgeführt wird.

18. Verfahren nach Anspruch 16, wobei die Inkubation des Proteins oder Peptids in Gegenwart der Phosphatase PP1 durchgeführt wird.

19. Verfahren nach Anspruch 16, wobei die Inkubation des Proteins oder Peptids in Gegenwart der Phosphatase PP4 (auch als PPX bezeichnet) oder PP6 (auch als PPV bezeichnet) durchgeführt wird.

20. Verfahren nach mindestens einem der Ansprüche 1 bis 15, wobei die Phosphatase eine Tyrosinphosphatase mit zweifacher Spezifität ist.

21. Verfahren nach Anspruch 20, wobei die Phosphatase mit zweifacher Spezifität CL100/3CH134 oder PAC1 oder hVH-2/MKP-2 oder hVH-3/B23 oder hVH-5 oder MKP-3/PYST1 oder B59 oder MKP-4 oder MKP-5 ist.

22. Verfahren nach mindestens einem der Ansprüche 1 bis 21, wobei das Protein oder Peptid an der Y-Position durch die Wirkung der Phosphatase dephosphoryliert wird.

23. Verfahren nach mindestens einem der Ansprüche 1 bis 22, wobei der Antikörper ein monoklonaler oder polyklonaler Antikörper ist.

24. Verfahren nach Anspruch 23, wobei der Antikörper ein polyklonaler Antikörper ist.

25. Verfahren nach Anspruch 23, wobei der Antikörper ein polyklonaler Antikörper ist, der für aktives JNK spezifisch ist.

26. Kit zum Nachweis von Phosphatase-Aktivität in einem Immunoassay, umfassend die folgenden Komponenten:
- eine Phosphatase;
- ein Substrat wie in Anspruch 1 definiert;
- einen Antikörper wie in Anspruch 1 definiert.

27. Kit nach Anspruch 26, wobei die Phosphatase eine Serin- oder Threoninphosphatase ist.

28. Kit nach Anspruch 26, wobei die Phosphatase eine Tyrosinphosphatase mit zweifacher Spezifität ist.

29. Verwendung des Assay-Verfahrens nach mindestens einem der Ansprüche 1 bis 25 oder des Kits nach mindestens einem der Ansprüche 26 bis 28 zum Screenen hinsichtlich von Modulatoren von Phosphatase-Aktivität, insbesondere Inhibitoren für eine Serin- oder Threoninphosphatase oder Phosphatase mit zweifacher Spezifität.

## Revendications

1. Processus de détection de l'activité de la phosphatase dans un dosage immunologique comprenant les étapes suivantes :
a) la fourniture d'une protéine ou d'un peptide comprenant le motif de séquence :
-Z-X-Y- ou -Y-X-Z-
dans lequel :
Z = sérine ou thréonine
X = une séquence de 1 à 10 acides aminés qui peuvent être identiques ou différents
Y = tyrosine, sérine ou thréonine
en tant qu'un substrat pour une phosphatase, ladite protéine ou ledit peptide étant pré-phosphorylé(e) aux positions Y et Z ;
b) incubation de la protéine ou du peptide avec une phosphatase pour former une protéine ou un peptide qui est déphosphorylé(e) à la position Z ;
c) l'addition d'un anticorps ayant une spécificité envers le peptide ou la protéine qui est phosphorylé(e) aux positions Y et Z ; et
d) la détection de l'activité de la phosphatase.

2. Processus selon la revendication 1, dans lequel le dosage immunologique est réalisé comme un dosage immunologique de liaison directe, de préférence un dosage immunologique de liaison directe et homogène.

3. Processus selon la revendication 2, dans lequel un peptide marqué ou une protéine marquée est utilisé(e).

4. Processus selon la revendication 2, dans lequel un anticorps marqué est utilisé.

5. Processus selon la revendication 3 ou 4, dans lequel le peptide/protéine ou l'anticorps est marqué par une étiquette luminescente, un marqueur radioactif, une enzyme marqueur ou un ligand d'affinité.

6. Processus selon la revendication 1, dans lequel le dosage immunologique est réalisé comme un dosage immunologique de liaison indirecte, de préférence comme un dosage immunologique de liaison indirecte et homogène.

7. Processus selon la revendication 6, dans lequel un ligand bis-phosphorylé marqué est ajouté pour être en compétition avec la protéine ou le peptide bis-phosphorylé(e) pour la liaison à l'anticorps, ledit ligand étant phosphorylé aux positions Y et Z.

8. Processus selon la revendication 7, dans lequel le ligand étant bis-phosphorylé aux positions Y et Z est marqué par une étiquette luminescente, un marqueur radioactif, une enzyme marqueur ou un ligand d'affinité.

9. Processus selon la revendication 8, dans lequel le ligand est 5-Tamra-AEEA-Lys-Phe-Met-Met-pThr-Pro-pTyr-Val-Val-Thr-Arg-NH₂, AEEA représentant un liant d'acide 8-amino-3,6-dioxaoctanoïque.

10. Processus selon au moins l'une des revendications 1 à 9, dans lequel le dosage est réalisé comme un dosage immunologique par fluorescence, particulièrement comme un dosage immunologique par polarisation de fluorescence, un dosage spectroscopique par corrélation de fluorescence, une mesure de la durée de vie de la fluorescence, ou une mesure de distribution de l'intensité fluorescente.

11. Processus selon au moins l'une des revendications 1 à 10, dans lequel X dans le motif de séquence comprend la proline ou le glutamate ou la glycine.

12. Processus selon au moins l'une des revendications 1 à 11, dans lequel la protéine fournie est la protéine JNK1, JNK2 ou JNK3 activée.

13. Processus selon au moins l'une des revendications 1 à 11, dans lequel le peptide fourni comprend des séquences identiques à celles de la boucle du site actif de JNK1, JNK2 ou JNK3 activée.

14. Processus selon les revendications 1 à 11, dans lequel le peptide comprend la séquence d'acides aminés H-Lys-Phe-Met-Met-pThr-Pro-pTyr-Val-Val-Thr-Arg-NH₂ (p signifie phosphorylé).

15. Processus selon les revendications 1 à 11, dans lequel le peptide se compose de la séquence d'acides aminés H-Lys-Phe-Met-Met-pThr-Pro-pTyr-Val-Val-Thr-Arg-NH₂ (p signifie phosphorylé).

16. Processus selon au moins l'une des revendications 1 à 15, dans lequel la phosphatase est une sérine ou une thréonine phosphatase.

17. Processus selon la revendication 16, dans lequel l'incubation de la protéine ou du peptide est réalisée en présence d'une protéine sérine/thréonine phosphatase de type 2A(PP2A) ou de type 2B (PP2B).

18. Processus selon la revendication 16, dans lequel l'incubation de la protéine ou du peptide est réalisée en présence de phosphatase PP1.

19. Processus selon la revendication 16, dans lequel l'incubation de la protéine ou du peptide est réalisée en présence de la phosphatase PP4 (aussi dénommée PPX) ou PP6 (aussi dénommée PPV).

20. Processus selon au moins l'une des revendications 1 à 15 dans lequel la phosphatase est une tyrosine phosphatase avec une double spécificité.

21. Processus selon la revendication 20, dans lequel la phosphatase à double spécificité est CL100/3CH134, ou PAC1, ou hVH-2/MKP-2, ou hVH-3/B23, ou hVH-5, ou MKP-3/PYST1, ou B59, ou MKP-4 ou MKP-5.

22. Processus selon au moins l'une des revendications 1 à 21, dans lequel la protéine ou le peptide est déphosphorylé(e) à la position Y par l'action de la phosphatase.

23. Processus selon au moins l'une des revendications 1 à 22, dans lequel l'anticorps est un anticorps monoclonal ou polyclonal.

24. Processus selon la revendication 23, dans lequel l'anticorps est un anticorps polyclonal.

25. Processus selon la revendication 23, dans lequel l'anticorps est un anticorps polyclonal spécifique de JNK active.

26. Kit de détection de l'activité de la phosphatase dans un dosage immunologique comprenant les composants suivants :
- une phosphatase ;
- un substrat tel que défini selon la revendication 1 ;
- un anticorps tel que défini selon la revendication 1.

27. Kit selon la revendication 26, dans lequel la phosphatase est une sérine ou une thréonine phosphatase.

28. Kit selon la revendication 26, dans lequel la phosphatase est une tyrosine phosphatase à double spécificité.

29. Utilisation du processus de dosage selon au moins l'une des revendications 1 à 25, ou kit selon au moins l'une des revendications 26 à 28, pour le criblage de modulateurs pour l'activité de la phosphatase, notamment des inhibiteurs pour une sérine ou une thréonine ou une phosphatase à double activité.
